# EUROPEAN PATENT SPECIFICATION

(11) **EP 1 496 126 B1**
(45) Date of publication and mention of the grant of the patent: **31.08.2005**
(21) Application number: 04016151.5
(22) Date of filing: 08.07.2004
(51) Int. Cl.: C12Q 1/00, G01N 33/52

(54) **Nanoparticles for optical sensors**
Nanopartikeln für optische Sensoren
Nanoparticules pour senseurs optiques

(30) Priority: 10.07.2003 EP 03015781
(43) Date of publication of application: 12.01.2005
(73) Proprietor: F. HOFFMANN-LA ROCHE AG, 4070 Basel (CH); Roche Diagnostics GmbH, 68305 Mannheim (DE)
(72) Inventor: Desprez, Valerie, 69115 Heidelberg (DE); Oranth, Norbert, 79106 Freiburg (DE); Spinke, Jürgen, 64653 Lorsch (DE); Tusa, James Kenneth, Alpharetta, GA 30004 (US)
(74) Representative: Dey, Michael

(56) References cited:
- WO-A-99/32662
- US-B1- 6 238 930

## Description

The present invention concerns a polymer layer for a sensor, wherein the polymer layer has nanoparticles embedded therein which impart to the polymer layer recognizing properties as well as transducer properties, a sensor comprising such layer and the use of the sensor for detecting or/and quantifying a target analyte.

Various sensors for determining substances of interest in qualitative and quantitative manner have been described to date. In particular, in the fields of environmental and food technology, medicine and biotechnology the development of precise analytical means and methods is of great interest. For example, enzyme-based sensors with electrochemical or optical transduction are widely used to determine analytes in the blood and in other body liquids.

Generally, classical sensors consist of a multilayer structure. For example, U.S. Patent No.6,107,083 describes an optical enzyme-based sensor with a multilayer structure which comprises, in the sequence of layers: (a) an oxygen-sensitive layer containing a luminescent dye in a light-transmissive, oxygen-permeable matrix,(b) an enzymatic layer containing an oxidative enzyme in a hydratable and oxygen-permeable matrix, and (c) a rapidly hydrating gas-permeable cover layer disposed over the enzymatic layer.

Major drawbacks of such multilayer-structured sensors are the complex buildup of the sensors involving problems with the coating compatibility of the multiple layers, the limited density of functional elements available on a planar surface and the difficulty in controlling precisely the thickness of each layer in order to maintain reproducible diffusion processes. These processes are accompanied by unsatisfactory sensor responses and reduced signal yields.

In U.S. Patent No.6,238,930 a different approach for a layer structure for the determination of a substance has been made by forming a micellar recognition system from two non-miscible phases, a surface-active substance and a recognition component and by incorporating this system into a layer. The substance of interest is then detected by the interaction with the recognition component and a transducing step both occurring in said layer. The construction of the layer structure according to U.S. 6,238,930 is complex and due to the requirement of forming appropriate micelles, the flexibility and field of application of such systems is limited. Compared to the classical multilayer-structured sensors no significant simplification could be achieved.

It is thus an object of the present invention to provide a sensor with simplified and reproducible sensor design which allows the precise determination of an analyte of interest.

The problem underlying the present invention is solved by a sensor comprising a polymer layer having recognizing and transducer properties, wherein these properties are provided by a recognizing component and a transducer contained in one or more nanoparticles embedded in the polymer layer.

According to the present invention, wherein the recognizing component and the transducer are provided in one single layer, the number of layers in the sensor are reduced, leading to a simplified sensor production. Moreover, by combining the recognizing component and the transducer in close vicinity in a unique layer, fast sensor responses can be achieved and the efficiency of the sensor is less dependent on the layer thickness, resulting in better coating reproducibility. In particular, by providing nanoparticles which impart to the layer recognizing and transducer properties the sensor system can be conveniently adjusted to different sensor applications by varying the constituents and design of the nanoparticles. For example, the size and density and arrangement of components can be varied. By that means, for example, it is possible to adjust the total amount of recognizing component or transducer in the polymer layer. In addition thereto, the use of functionalized nanoparticles in the polymer layer according to the invention allows to adjust the sensitivity and dynamic range by changing the density of nanoparticles in the polymer layer. A further advantage of the sensor according to the invention is that it is suitable for performing multiple measurements due to easy regeneration of the polymer layer.

The improved sensor according to the invention is suitable for use in the fields of pharmaceutics, medicine, biotechnology, environmental and food technology and drinking and waste water control. The sensor according to the invention, therefore, is brought together with the sample containing the substance to be detected leading to an interaction between the analyte and the recognizing component in the polymer layer. The interaction between recognizing component and analyte results in a product or a change in the conditions in the polymer layer. In the following this interaction will be termed the recognizing step. In close contact to the recognizing component the transducer is provided which consists of a component which is sensitive to a product or change in conditions resulting from the recognizing step. "Sensitivity" to the product or the change in conditions means that the transducer properties are changed by the influence of said product or said different ambient conditions, wherein the change in properties of the transducer can be detected.

Sensors based on the reaction of an oxidative enzyme for the detection of an oxidizable substance are particularly useful. In the enzymatic oxidation oxygen in the environment of the enzyme is consumed, leading to a change in the oxygen concentration in the polymer layer which is directly connected with the amount of analyte in the sample. The change of the oxygen concentration can be made visible, for example, by using an oxygen-sensitive luminescent dye. By means of such dye the amount of analyte in the sample can be detected by measuring the change in the luminescence of the dye. In general, luminescence is quenched by oxygen, i.e. due to the consumption of oxygen, the enzymatic reaction leads to a stronger luminescence and the luminescence intensity change can be detected as a function of the amount of analyte in the sample.

The combination of recognizing and transducer properties in one single polymer layer in the sensor according to the present invention is realized by embedding functionalized nanoparticles into the polymer layer. Two types of functionalized nanoparticles can be used: Particles comprising a recognizing component and a transducer and nanoparticles each comprising only one of the two components. These nanoparticles can be combined as desired, but must be chosen such that the polymer layer exhibits recognizing as well as transducer properties.

In one preferred embodiment of the invention the polymer layer of the sensor has embedded therein first nanoparticles comprising a recognizing component and second nanoparticles comprising a transducer.

In a second preferred embodiment of the present invention the polymer layer of the sensor has embedded therein nanoparticles comprising both a recognizing component and a transducer.

According to the present invention the recognizing component is a chemical or biological substance which is capable of selectively interacting with an analyte in a recognizing step. Through this interaction the analyte can be detected or/and quantified. For example, the recognizing step is a reaction or coupling between the recognizing component and the analyte, leading to a detectable consumption of a substance, the formation of a detectable product or to a detectable change of the ambient conditions.

Preferably, the recognizing component is a bioactive component or living cells or bacteria. In particular, the bioactive component is selected from the group consisting of enzymes, synthetic and gene-manipulated enzymes and antibodies, peptides, carbohydrates, lectins, lipids and mixtures thereof. More preferred, the bioactive component is an enzyme which can be selected from hydrolases, proteases and oxidases, depending on the substance to be detected. Oxidative enzymes are particularly suitable. For example, analytes which are enzyme-oxidizable such as glucose, cholesterol, lactate or sarcosine can be detected by using oxidative enzymes. The oxidative enzyme may be selected from the group consisting of glucose oxidase, cholesterol oxidase, lactate oxidase, sarcosine oxidase and mixtures thereof.

According to the invention the recognizing component can also consist of several chemical substances or/and biological substances, leading to a cascade recognizing system. This is particularly useful when the analyte of interest does not directly result in a detectable and quantifiable signal, respectively, by interaction with the recognizing component. Here, a second or further recognizing component reacts or interacts with the product of one of the foregoing recognizing steps to result in an appropriate product or change in the ambient conditions and to produce a signal.

The transducer according to the invention consists of a component which is sensitive to a component of the recognizing step or to a change of the ambient conditions caused by said step. It can be an optical or electrochemical transducer, i.e. the resulting measurement variable is an optical signal and an electrical signal, respectively.

An optical transducer consists of a component which is capable of changing its spectral properties in dependence on the change of the ambient conditions caused by the recognizing step. The properties may be transformed by change of the pH, the presence of specific ions or molecules, oxidizing agents or reducing agents. For example, a change in intensity of the radiation emitted from the optical transducer can be influenced by a compound consumed or produced in the recognizing step and can be correlated with the presence or/and amount of the substance of interest. Such a change in intensity can be due to quenching of the emission, for example, by oxygen. Either by detecting a decrease or increase in luminescence intensity - depending on whether the recognizing step produces or consumes oxygen - information about the type or/and amount of analyte can be achieved. Generally, the quenching of emitted radiation, i.e. the presence of oxygen, leads to a decrease, while an increase is detected when oxygen is consumed. Further, the optical transducer can also consist of a substance which at first does not produce an optical signal but which is transformed by a product or by means of a change of the ambient conditions resulting from the recognizing step to produce an optical signal.

An electrochemical transducer, for example, reacts to a change of the current, potential or conductivity caused by the recognizing step.

In a preferred embodiment of the present invention the transducer in the sensor is an optical transducer. Preferably, the optical transducer consists of a dye which is sensitive to a component which is consumed or formed in the recognizing step. More preferred is the use of a luminescent dye. Suitable dyes for use in the sensor of the present invention are selected from the group consisting of ruthenium(II), osmium(II), iridium(III), rhodium(III) and chromium(III) ions complexed with 2,2'-bipyridine, 1,10-phenanthroline, 4,7-diphenyl-1,10-phenanthroline, 4,7-dimethyl-1,10-phenanthroline, 4,7-disulfonated-diphenyl-1,10-phenanthroline, 5-bromo-1,10-phenanthroline, 5-chloro-1,10-phenathroline, 2,2'-bi-2-thiazoline, 2,2'-dithiazole, VO²⁺, Cu²⁺, Zn²⁺, Pt²⁺ Pd²⁺ complexed with porphyrin, chlorine and phthalocyanine and mixtures thereof. In a preferred embodiment the luminescent dye is [Ru(diphenylphenantroline)3], octaethyl-Pt-porphyrin, octaethyl-Pt-porphyrin ketone, tetrabenzo-Pt-porphyrin, tetraphenyl-Pt-porphyrinmeso-tetraphenyl-tetrabenzo-Pt-porphyrin, tetracyclohexenyl-Pt-porphyrin, octaethyl-Pt-chlorine, tetraphenyl-Pt-chlorine or a mixture thereof.

Further possible transducers, for example, contain indicators such as redox indicators which lead to an optical signal by the oxidation/reduction of the indicator, pH indicators responding to a change of the proton concentration and ionophores or other suitable chelating agents which form optically detectable chelates or complexes with ions or/and molecules formed in the recognizing step.

The nanoparticles comprising the recognizing component or/and the transducer preferably are solid or semi-solid particles. More preferred is the use of solid materials such as solid polymeric materials. The material of the solid or semi-solid particles generally may be selected from any inorganic or organic synthetic or natural material. Preferably, the nanoparticles are made from polymeric materials or semiconductor materials (e.g. so-called quantum dots). Examples of suitable inorganic materials are SiO₂, BaSO₄, glass etc. Organic materials may be selected from synthetic and natural polymers. Examples of natural polymers are alginates, cellulose or cellulose derivatives.

Specific examples of suitable polymers are polyolefins, vinyl polymers, polyamides, polyesters, polyacetals, polycarbonates, polyurethanes, polysiloxanes and copolymers and mixtures thereof. In particular, the nanoparticles are made of polystyrene, poly(tert-butylstyrene), polyethylene, polypropylene, polybutene, polyisobutene and copolymers and mixtures thereof.

With oxidase enzymes as recognizing component, the nanoparticles are preferably made from polymers or co-polymers having a high oxygen permeability. Specific examples of suitable polymers are silicone, polybutadiene, poly-(tert.-butyl styrene).

The nanoparticles may comprise components to modify physical properties (e.g., density, reflective index) and the properties of active components. The size of the nanoparticles can vary from 10 to 500 nm of mean particle diameter. Preferably, the mean diameter of the nanoparticles is 30 to 300 nm, more preferred 100 to 200 nm. The size of the particles, for example, depends on the amount of active components to be incorporated in the nanoparticle or on the desired density of active components in the nanoparticles.

The recognizing component or/and the transducer is entrapped within, conjugated to or attached to the nanoparticles or the nanoparticle material, respectively.

For example, the recognizing component or/and the transducer are entrapped within the nanoparticle, i.e. they are not covalently bound to the nanoparticle material, but only held therein by physical entrapment. In particular, the active components are homogeneously distributed therein. It is also possible to conjugate the recognizing component or/and the transducer to the nanoparticle material at the surface of the nanoparticle or/and inside the nanoparticle. Conjugation is due to intermolecular forces such as electrostatic interaction, induction forces and hydrogen bondings or ionic bonding. It is further possible to attach, i.e. covalently bind, said components onto the surface of the nanoparticles or/and to the nanoparticle material within the nanoparticles.

In a preferred embodiment of the invention the transducer is entrapped within the nanoparticle and the recognizing component is covalently attached to the surface of the nanoparticle.

In another preferred embodiment of the invention the nanoparticle comprises both the transducer and the recognizing component, wherein the transducer is entrapped within the nanoparticle and the recognizing component is covalently attached to the surface of the nanoparticle.

In the sensor according to the invention the nanoparticles as described above are embedded in a polymer layer to impart to the layer recognizing as well as transducer properties. Embedment is accomplished by physical entrapment or/and covalent linking or/and conjugation of the nanoparticles to the polymer layer. Preferably, the nanoparticles are dispersed in the polymer layer and are held therein by physical entrapment.

The nanoparticles can be homogeneously distributed in the polymer layer or it may be desired to have different densities of nanoparticles within the polymer layer. For example, it may be preferred to have more nanoparticles near the contacting surface of the sensor than inside or at the other end of the layer.

In general, the polymer layers can be made from any inorganic or organic natural or synthetic polymer, wherein it is preferred that the polymer is rapidly hydratable. Further it may be preferred that the polymer layer is oxygen-permeable, in particular, if oxygen is involved in the transducing step.

Examples of suitable polymers for the polymer layer in the sensor according to the invention preferably are selected from the group consisting of polyolefins, vinyl polymers, polyamides, polyesters, polyacetals, polycarbonates, polyurethanes and copolymers and mixtures thereof. The polymer layer preferably consists of one or more polyurethanes.

An advantage of the sensor according to the invention is that the sensitivity and dynamic range of the sensor can be adjusted by varying the density of nanoparticles in the layer and by varying the amount of recognizing component or/and transducer in each nanoparticle. Thus, for example, by having a high density of nanoparticles in the layer, fast sensor responses can be achieved.

A specific embodiment of the present invention is a sensor comprising (a) a light-transmissive substrate,(b) a polymer layer having recognizing and optical transducer properties, wherein these properties are provided by a recognizing component and an optical transducer contained in the polymer layer deposited on (a), and (c) a membrane layer on top of the polymer layer.

The light-transmissive substrate in this embodiment of the invention should be transmissive to radiation for exciting the optical transducer, which preferably is a luminescent dye. Additionally, the radiation emitted from the transducer must pass the light-transmissive substrate in the opposite direction for detection. Preferably, the light-transmissive substrate should have a low permeability to gas, in particular, oxygen. In particular, when using a transducer which is sensitive to gas, in particular, oxygen-sensitive, distortion of the response of the transducer can be avoided thereby. Suitable materials for the light-transmissive substrate are organic or inorganic materials, in particular polymeric materials, e.g. glass, in particular, MYLAR™ glass, polyethylene terephthalate (PET) and polyvinylidene chloride (PVO).

On this layer, a polymer layer as described above is deposited and a membrane layer (c) covers the polymer layer.

The membrane layer preferably provides optical isolation and is coated on the polymer layer for adjustment of diffusional processes. It can be constructed such that only certain substances can pass, for example, the analyte to be detected, and preferably is rapidly hydratable.

Possible materials for the membrane layer are non-water soluble polymers and preferably from polyurethane, polyacrylamide, polystyrene, polyvinyl esters and co-polymers of, e.g., butadiene and styrene. Preferably, carbon black is incorporated in the membrane layer for optical isolation.

A preferred embodiment according to the invention is a sensor for the detection of glucose in a sample. In this embodiment the recognizing component is the oxidative enzyme glucose oxidase and the optical transducer is a luminescent dye. Glucose present in the sample is oxidized by glucose oxidase and oxygen, consuming oxygen in the polymer layer. The transducer consisting of a luminescent dye which is oxygen-sensitive responds to the depletion of oxygen by increasing the luminescence intensity which can be detected spectroscopically.

A further advantage of the sensor according to the invention is that it can be fast regenerated. For example, if used as an oxidative enzyme-based sensor, the oxygen concentration inside the polymer layer of the sensor, which was consumed during the enzymatic reation with the target analyte, can be fast regenerated.

Another specific embodiment of the invention is a sensor which is suitable for the detection of analytes which cannot be enzymatically oxidized in a single step such as creatinine. In this case the recognizing component consists of several enzymes and creatinine is converted into an oxidizable intermediate (sarcosine) by a first enzyme, which then can be oxidized by an oxidative enzyme (sarcosine oxidase). The consumption of oxygen in the enzymatic oxidation can be detected by an oxygen-sensitive dye, analogous to the glucose sensor described above.

The sensor according to the invention is very useful for qualitatively or quantitatively analyzing a sample such as a body liquid sample.

Consequently, a further embodiment of the present invention is the use of a sensor according to the invention for detecting or/and quantifying a target analyte. For example, the sensor according to the invention can be used for the detection of a specific substance in environmental, food, waste water, drinking water and medicinal samples or for analysis in the fields of biotechnology. The inventive sensor is particularly useful for medicinal applications such as the detection or/and quantification of target analytes in body liquid samples such as blood or serum, urine and saliva in pure or diluted form. In particular, the target analyte of interest is selected from the group consisting of glucose, lactate, sarcosine, creatinine and mixtures thereof, the sensor being particularly useful for detecting or/and quantifying glucose.

A further embodiment of the present invention is a polymer layer having recognizing and transducer properties, wherein these properties are provided by a recognizing component and a transducer contained in one or more nanoparticles embedded in the polymer layer.

Specific examples of suitable polymeric materials, transducers and recognizing components are given above.

The polymer layer is particularly useful for incorporation in a sensor as described above.

Still another embodiment of the present invention is a process for the production of a sensor comprising a polymer layer with recognizing and transducer properties, the process comprising the steps of (a) providing a light-transmissive substrate, (b) depositing on said substrate a polymer layer having recognizing and transducer properties, wherein these properties are provided by a recognizing component and a transducer contained in one or more nanoparticles embedded in the polymer layer, and (c) coating a membrane layer on top of said polymer layer.

Preferably, the polymer layer according to step (b) is coated with a membrane layer incorporating carbon black (step (c). This coating provides optical isolation and adjustment of diffusional processes.

Still another embodiment of the invention is the use of a nanoparticle comprising a recognizing component or/and a transducer in a sensor or a polymer layer according to the invention.

The invention will be further illustrated by the following Figures and Examples.

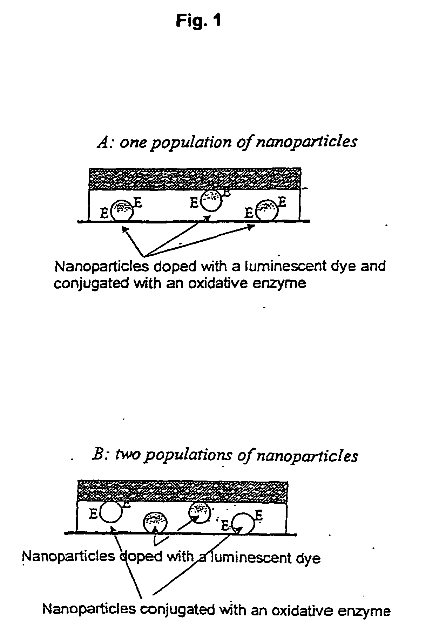

Fig.1 is a schematic drawing illustrating two preferred embodiments of the present invention. (A) represents the inventive polymer layer comprising nanoparticles doped with a luminescent dye and conjugated with an oxidative enzyme. (B) represents an embodiment of the invention, where two populations of nanoparticles are embedded in the polymer layer, nanoparticles doped with a luminescent dye and nanoparticles conjugated with an oxidative enzyme.

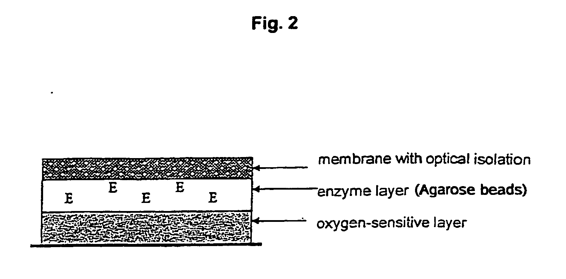

Fig.2 shows a conventional multilayer-structured sensor, wherein the recognizing component (enzyme) and the transducer (oxygen-sensitive) are contained in separate layers.

### Example 1

A glucose sensor is constructed according to Fig.1B and contains 30 mg of poly(tert-butylstyrene) nanoparticles doped with a ruthenium complex [Ru(diphenylphenanthroline)₃] and 50 mg of polystyrene nanoparticles conjugated with glucose oxidase dispersed in a polyurethane matrix.

On top of this multifunctional layer, a membrane incorporating carbon black is then coated for optical isolation and for the adjustment of diffusional processes.

The Table below illustrates the fluorescence signal response [kinetic measurements: fluorescence intensity change per second (ΔI per second)] to various levels of glucose in control solutions tonometered at 150 Torr oxygen partial pressure.

| Glucose concentration (mg/dL) | Relative slope (ΔI per second) |
|---|---|
| 50 | 727 |
| 113 | 1451 |
| 356 | 3752 |

After injection of each control solution the cassette was washed manually 5 times with the Opti buffer tonometered at 90 Torr of oxygen before being flushed externally with a gas containing 90 Torr oxygen for the recalibration (recalibration times were less than 60 s).

## Claims

1. Sensor comprising a polymer layer having recognizing and transducer properties, wherein these properties are provided by a recognizing component and a transducer contained in one or more nanoparticles embedded in the polymer layer.

2. Sensor according to claim 1, the polymer layer having embedded therein first nanoparticles comprising a recognizing component and second nanoparticles comprising a transducer.

3. Sensor according to claim 1, the polymer layer having embedded therein nanoparticles comprising both a recognizing component and a transducer.

4. Sensor according to one of the preceding claims, wherein the recognizing component is a bioactive component.

5. Sensor according to claim 4, wherein the bioactive component is selected from the group consisting of enzymes, synthetic and gene-manipulated enzymes.

6. Sensor according to claim 5, wherein the bioactive component is an oxidative enzyme selected from the group consisting of glucose oxidase, cholesterol oxidase, lactate oxidase, sarcosine oxidase and mixtures thereof.

7. Sensor according to claim 6, wherein the oxidative enzyme is glucose oxidase.

8. Sensor according to one of the preceding claims, wherein the transducer is an optical transducer.

9. Sensor according to claim 8, wherein the optical transducer consists of a luminescent dye.

10. Sensor according to claim 9, wherein the luminescent dye is selected from the group consisting of [Ru(diphenylphenantroline)₃], octaethyl-Pt-porphyrin, octaethyl-Pt-porphyrin ketone, tetrabenzo-Pt-porphyrin, tetraphenyl-Pt-porphyrin, meso-tetraphenyl-tetrabenzo-Pt-porphyrin, tetracylcohexenyl-Pt-porphyrin, octaethyl-Pt-chlorine, tetraphenyl-Pt-chlorine and mixtures thereof.

11. Sensor according to one of the preceding claims, wherein the nanoparticles are polymer nanoparticles.

12. Sensor according to claim 11, wherein the polymer material of the nanoparticles is selected from the group consisting of polyolefins, vinyl polymers, polyamides, polyesters, polyacetals, polycarbonates, polyurethanes, polysiloxanes and copolymers and mixtures thereof.

13. Sensor according to one of the preceding claims, wherein the mean diameter of the nanoparticles is 10 to 500 nm.

14. Sensor according to one of the preceding claims, wherein the polymer layer consists of a polymer selected from the group consisting of polyolefins, vinyl polymers, polyamides, polyesters, polyacetals, polycarbonates, polyurethanes and copolymers and mixtures thereof.

15. Sensor according to claim 14, wherein the polymer layer consists of one or more polyurethanes.

16. Sensor according to one of the preceding claims, wherein the recognizing component and the transducer are entrapped within, conjugated to or attached to one or more nanoparticles embedded in the polymer layer.

17. Sensor according to one of the preceding claims, comprising
(a) a light-transmissive substrate,
(b) a polymer layer having recognizing and optical transducer properties, wherein these properties are provided by a recognizing component and an optical transducer contained in one or more nanoparticles embedded in the polymer layer deposited on (a), and
(c) a membrane layer on top of the polymer layer.

18. Use of a sensor according to one of the preceding claims for detecting or/and quantifying a target analyte.

19. Use according to claim 18, wherein the target analyte is selected from the group consisting of glucose, lactate, sarcosine, creatinine and mixtures thereof.

20. Use according to claim 19, wherein the target analyte is glucose.

21. Use according to one of claims 18-20 where a target analyte in a body liquid sample is detected or/and quantified.

22. Polymer layer having recognizing and transducer properties, wherein these properties are provided by a recognizing component and a transducer contained in one or more nanoparticles embedded in the polymer layer.

23. Polymer layer according to claim 22, wherein the recognizing component is a bioactive component.

24. Polymer layer according to claim 22 or 23, wherein the transducer is an optical transducer.

25. Process for the production of a sensor comprising a polymer layer with recognizing and transducer properties, the process comprising the steps of
(a) providing a light-transmissive substrate,
(b) depositing on said substrate a polymer layer according to one of claims 22-24, and
(c) coating a membrane layer on top of said polymer layer.

26. Process according to claim 25, wherein the membrane layer has carbon black incorporated therein.

27. Use of a nanoparticle comprising a recognizing component or/and a transducer in a sensor according to one of claims 1-17 or a polymer layer according to one of claims 22-24.

## Patentansprüche

1. Sensor, umfassend eine Polymerschicht mit erkennenden und Transducer-Eigenschaften, wobei diese Eigenschaften durch einen erkennenden Bestandteil und einen Transducer bereitgestellt werden, die in einem oder mehreren, in die Polymerschicht eingebetteten Nanopartikeln enthalten sind.

2. Sensor nach Anspruch 1, wobei in der Polymerschicht erste Nanopartikel, die einen erkennenden Bestandteil umfassen, und zweite Nanopartikel, die einen Transducer umfassen, eingebettet sind.

3. Sensor nach Anspruch 1, wobei in der Polymerschicht Nanopartikel eingebettet sind, die sowohl einen erkennenden Bestandteil als auch einen Transducer umfassen.

4. Sensor nach einem der vorhergehenden Ansprüche, wobei der erkennende Bestandteil ein biologisch aktiver Bestandteil ist.

5. Sensor nach Anspruch 4, wobei der biologisch aktive Bestandteil ausgewählt ist aus der Gruppe bestehend aus Enzymen, synthetischen und genmanipulierten Enzymen.

6. Sensor nach Anspruch 5, wobei der biologisch aktive Bestandteil ein oxidatives Enzym ist, das ausgewählt ist aus der Gruppe bestehend aus Glucoseoxidase, Cholesterinoxidase, Lactatoxidase, Sarcosinoxidase und Gemischen davon.

7. Sensor nach Anspruch 6, wobei das oxidative Enzym Glucoseoxidase ist.

8. Sensor nach einem der vorhergehenden Ansprüche, wobei der Transducer ein optischer Transducer ist.

9. Sensor nach Anspruch 8, wobei der optische Transducer aus einem lumineszierenden Farbstoff besteht.

10. Sensor nach Anspruch 9, wobei der lumineszierende Farbstoff ausgewählt ist aus der Gruppe bestehend aus [Ru(diphenylphenantrolin)₃], Octaethyl-Pt-porphyrin, Octaethyl-Pt-porphyrinketon, Tetrabenzo-Pt-porphyrin, Tetraphenyl-Pt-porphyrin, Meso-tetraphenyl-tetrabenzo-Pt-porphyrin, Tetracyclohexenyl-Pt-porphyrin, Octaethyl-Pt-chlor, Tetraphenyl-Pt-chlor und Gemischen davon.

11. Sensor nach einem der vorhergehenden Ansprüche, wobei die Nanopartikel Polymernanopartikel sind.

12. Sensor nach Anspruch 11, wobei das Polymermaterial der Nanopartikel ausgewählt ist aus der Gruppe bestehend aus Polyolefinen, Vinylpolymeren, Polyamiden, Polyestern, Polyacetalen, Polycarbonaten, Polyurethanen, Polysiloxanen und Copolymeren und Gemischen davon.

13. Sensor nach einem der vorhergehenden Ansprüche, wobei der mittlere Durchmesser der Nanopartikel 10 bis 500 nm beträgt.

14. Sensor nach einem der vorhergehenden Ansprüche, wobei die Polymerschicht aus einem Polymer besteht, welches ausgewählt ist aus der Gruppe bestehend aus Polyolefinen, Vinylpolymeren, Polyamiden, Polyestern, Polyacetalen, Polycarbonaten, Polyurethanen und Copolymeren und Gemischen davon.

15. Sensor nach Anspruch 14, wobei die Polymerschicht aus einem oder mehreren Polyurethanen besteht.

16. Sensor nach einem der vorhergehenden Ansprüche, wobei der erkennende Bestandteil und der Transducer innerhalb eines oder mehrerer Nanopartikel eingeschlossen sind, an einen oder mehrere Nanopartikel konjugiert sind oder an einen oder mehrere Nanopartikel angebracht sind, welche in der Polymerschicht eingebettet sind.

17. Sensor nach einem der vorhergehenden Ansprüche, umfassend
(a) ein lichtdurchlässiges Substrat,
(b) eine Polymerschicht mit erkennenden und optischen Transducer-Eigenschaften, wobei diese Eigenschaften durch einen erkennenden Bestandteil und einen optischen Transducer bereitgestellt werden, die in einem oder mehreren Nanopartikeln in der Polymerschicht, die auf (a) aufgebracht ist, eingebettet sind, und
(c) eine Membranschicht auf der Polymerschicht.

18. Verwendung eines Sensors nach einem der vorhergehenden Ansprüche für den Nachweis oder/und die Quantifizierung eines Zielanalyten.

19. Verwendung nach Anspruch 18, wobei der Zielanalyt ausgewählt ist aus der Gruppe bestehend aus Glucose, Lactat, Sarcosin, Creatinin und Gemischen davon.

20. Verwendung nach Anspruch 19, wobei der Zielanalyt Glucose ist.

21. Verwendung nach einem der Ansprüche 18 bis 20, wobei ein Zielanalyt in einer Körperflüssigkeitsprobe nachgewiesen oder/und quantifiziert wird.

22. Polymerschicht mit erkennenden und Transducer-Eigenschaften, wobei diese Eigenschaften durch einen erkennenden Bestandteil und einen Transducer bereitgestellt werden, welche in einem oder mehreren Nanopartikeln enthalten sind, die in der Polymerschicht eingebettet sind.

23. Polymerschicht nach Anspruch 22, wobei der erkennende Bestandteil ein biologisch aktiver Bestandteil ist.

24. Polymerschicht nach Anspruch 22 oder 23, wobei der Transducer ein optischer Transducer ist.

25. Verfahren zur Herstellung eines Sensors, umfassend eine Polymerschicht mit erkennenden und Transducer-Eigenschaften, wobei das Verfahren die Schritte umfasst
(a) Bereitstellen eines lichtdurchlässigen Substrats,
(b) Aufbringen einer Polymerschicht nach einem der Ansprüche 22 bis 24 auf das Substrat und
(c) Beschichten der Oberseite der Polymerschicht mit einer Membranschicht.

26. Verfahren nach Anspruch 25, wobei in die Membranschicht Ruß eingebracht ist.

27. Verwendung eines Nanopartikels, der einen erkennenden Bestandteil oder/und einen Transducer in einem Sensor nach einem der Ansprüche 1 bis 17 oder eine Polymerschicht nach einem der Ansprüche 22 bis 24 umfasst.

## Revendications

1. Capteur comprenant une couche polymère ayant des propriétés de reconnaissance et de transducteur, dans lequel ces propriétés sont fournies par un composant de reconnaissance et un transducteur contenu dans une ou plusieurs nanoparticules enrobées dans la couche polymère.

2. Capteur selon la revendication 1, des premières nanoparticules comprenant un composant de reconnaissance et des secondes nanoparticules comprenant un transducteur étant enrobées dans la couche polymère.

3. Capteur selon la revendication 1, des nanoparticules comprenant à la fois un composant de reconnaissance et un transducteur étant enrobées dans la couche polymère.

4. Capteur selon l'une des revendications précédentes, dans lequel le composant de reconnaissance est un composant bioactif.

5. Capteur selon la revendication 4, dans lequel le composant bioactif est choisi dans le groupe constitué d'enzymes, d'enzymes synthétiques et manipulées génétiquement.

6. Capteur selon la revendication 5, dans lequel le composant bioactif est une enzyme oxydative choisie dans le groupe constitué de la glucose oxydase, cholestérol oxydase, lactase oxydase, sarcosine oxydase et leurs mélanges.

7. Capteur selon la revendication 6, dans lequel l'enzyme oxydative est la glucose oxydase.

8. Capteur selon l'une des revendications précédentes, dans lequel le transducteur est un transducteur optique.

9. Capteur selon la revendication 8, dans lequel le transducteur optique est constitué d'un colorant luminescent.

10. Capteur selon la revendication 9, dans lequel le colorant luminescent est choisi dans le groupe constitué de [Ru(diphénylphénantroline)₃], octaéthyl-Pt-porphyrine, octaéthyl-Pt-porphyrine cétone, tétrabenzo-Pt-porphyrine, tétraphényl-Pt-porphyrine, méso-tétraphényl-tétrabenzo-Pt-porphyrine, tétracyclohexényl-Pt-porphyrine, octaéthyl-Pt-chlore, tétraphényl-Pt-chlore et leurs mélanges.

11. Capteur selon l'une des revendications précédentes, dans lequel les nanoparticules sont des nanoparticules de polymère.

12. Capteur selon la revendication 11, dans lequel le matériau polymère des nanoparticules est choisi dans le groupe constitué des polyoléfines, polymères de vinyle, polyamides, polyesters, polyacétals, polycarbonates, polyuréthanes, polysiloxanes et des copolymères et mélanges de ceux-ci.

13. Capteur selon l'une des revendications précédentes, dans lequel le diamètre moyen des nanoparticules est de 10 à 500 nm.

14. Capteur selon l'une des revendications précédentes, dans lequel la couche polymère est constituée d'un polymère choisi dans le groupe constitué des polyoléfines, polymères de vinyle, polyamides, polyesters, polyacétals, polycarbonates, polyuréthanes, polysiloxanes et des copolymères et mélanges de ceux-ci.

15. Capteur selon la revendication 14, dans lequel la couche polymère est constituée d'un ou plusieurs polyuréthanes.

16. Capteur selon l'une des revendications précédentes, dans lequel le composant de reconnaissance et le transducteur sont enrobés dans, conjugués ou liés à une ou plusieurs nanoparticules enrobées dans la couche polymère.

17. Capteur selon l'une des revendications précédentes, comprenant
(a) un substrat transmettant la lumière,
(b) une couche polymère ayant des propriétés de reconnaissance et de transducteur optique, dans laquelle ces propriétés sont fournies par un composant de reconnaissance et un transducteur optique contenus dans une ou plusieurs nanoparticules enrobées dans la couche polymère déposée sur (a), et
(c) une couche de membrane sur la couche polymère.

18. Utilisation d'un capteur selon l'une des revendications précédentes pour détecter et/ou quantifier un analyte cible.

19. Utilisation selon la revendication 18, dans laquelle l'analyte cible est choisi dans le groupe constitué de glucose, lactate, sarcosine, créatinine et leurs mélanges.

20. Utilisation selon la revendication 19, dans laquelle l'analyte cible est le glucose.

21. Utilisation selon l'une des revendications 18 à 20 dans laquelle un analyte cible est détecté et/ou quantifié dans un échantillon de liquide corporel.

22. Couche polymère ayant des propriétés de reconnaissance et de transducteur, dans laquelle ces propriétés sont fournies par un composant de reconnaissance et un transducteur contenu dans une ou plusieurs nanoparticules enrobées dans la couche polymère.

23. Couche polymère selon la revendication 22, dans laquelle le composant de reconnaissance est un composant bioactif.

24. Couche polymère selon la revendication 22 ou 23, dans laquelle le transducteur est un transducteur optique.

25. Procédé pour la production d'un capteur comprenant une couche polymère avec des propriétés de reconnaissance et de transducteur, le procédé comprenant les étapes de
(a) fourniture d'un substrat transmettant la lumière,
(b) dépôt sur ledit substrat d'une couche polymère selon l'une des revendications 22 à 24, et
(c) dépôt d'une couche de membrane sur ladite couche polymère.

26. Procédé selon la revendication 25, dans lequel du noir de carbone est incorporé dans la couche de membrane.

27. Utilisation d'une nanoparticule comprenant un composant de reconnaissance et/ou un transducteur dans un capteur selon l'une des revendications 1 à 17 ou d'une couche polymère selon l'une des revendications 22 à 24.
